# EUROPEAN PATENT SPECIFICATION

(11) **EP 2 753 246 B1**
(45) Date of publication and mention of the grant of the patent: **07.11.2018**
(21) Application number: 12756222.1
(22) Date of filing: 10.09.2012
(51) Int. Cl.: A61B 17/00, A61B 17/12

(54) **A COLLAPSIBLE MEDICAL CLOSING DEVICE AND A METHOD OF ASSEMBLING THE DEVICE**
FALTBARE MEDIZINISCHE VERSCHLUSSVORRICHTUNG UND VERFAHREN ZUM AUFBAU DER VORRICHTUNG
DISPOSITIF DE FERMETURE MÉDICALE RÉTRACTABLE ET PROCÉDÉ DE MONTAGE DU DISPOSITIF

(30) Priority: 09.09.2011 EP 11180831; 13.09.2011 US 201161533848 P
(43) Date of publication of application: 16.07.2014
(73) Proprietor: Occlutech Holding AG, 8201 Schaffhausen (CH)
(72) Inventor: SOLEM, Jan Otto, 237 42 Bjärred (SE)
(74) Representative: KIPA AB
(86) International application number: PCT/EP2012/067649
(87) International publication number: WO 2013/034764

(56) References cited:
- EP-A1- 1 982 655
- WO-A1-2008/055301
- WO-A1-2008/074027
- WO-A1-2010/029190
- WO-A2-2011/093941
- US-A1- 2004 060 563
- US-A1- 2005 033 409
- US-A1- 2006 224 183
- US-A1- 2008 262 518
- US-B1- 6 936 055

## Description

### Field of the Invention

This disclosure pertains in general to the sealing of an opening in a body, such as a vessel or the wall of a heart cavity. More particularly the disclosure relates to a collapsible medical closing device for closing a body opening, a method of assembling a medical system outside a body before use and a system for delivering an object through a body opening to a target site in a body, as well as related systems and methods.

### Background of the Invention

The present disclosure is related to the sealing of an opening in a body vessel or the wall of a heart cavity, e.g. a blood vessel or a human heart, and more precisely to a device, a system and a method for performing such sealing. Heart diseases, e.g. coronary artery disease, heart valve disease and congenital heart disease are responsible for a majority of mortality among humans. Previously, almost all patients underwent open heart surgery in order to correct such disorders. In an increasing number of cases, the disorder is nowadays corrected by means of percutaneous catheter minimal invasive based therapy. However, to get access to the body vasculature and the heart cavities, catheters, sometimes of large diameters are passed through the walls of such cavities or vessels, and thus making holes.

Often such holes and channels dilated to considerably diameters, sometimes up to 7 to 9 mm, e.g. when inserting a heart valve by means of catheter techniques. While withdrawing such catheters, defects or holes remain open, threatening the patient to suffer from serious bleedings, sometimes life threatening. The largest channels have to be closed by surgical interventions, while the smallest in smaller vessels are left to close by nature. The latter means that a clot has to be formed in the channels by blood platelets and coagulation factors from the blood itself. To allow this process, compression from the outside is mandatory, sometimes for hours. Most used sealing methods involve some type of suturing to close the defect.

A device and a method for sealing punctures and incisions without the use of suturing are described in US Patent No 5,413,571. A biodegradable, fluid filled balloon that is positioned in the puncture seals the puncture. The device includes a shaft member that is left to be resorbed inside the body. The shaft member may be sutured to the skin or slightly below the outer surface of the skin.

Yet another device for sealing a puncture is described in U.S. Patent No. 5,916,236. The device comprises a retaining element having a distal occlusion element. The retaining element and thereby the occlusion element is fixed in the puncture by a fixing element positioned outside the vessel, and the three elements are left in the body to be resorbed.

A sealing device and method is described in SE2990827. Here the sealing member is an elongated flexible thin walled tubing. This tubing has an elastic reinforcement at its distal end, which is adapted to form a funnel-shaped sealing against the inner wall of a body vessel when the tubing is circumferentially pursed outside the body vessel.

From EP 1982 655 A1, an atrial appendage occlusion device is known. The occlusion device comprises a mesh or a braiding of at least one wire or thread. The occlusion device has been given a shape using a reshaping and/or heat-treatment process, and is self-expandable, as well as configured for safe anchoring in an atrial appendage of the left or right atrium of a heart, comprising proximal retention region at a proximal end of the occlusion device; a distal retention region; and a central region between the proximal retention region and the distal retention region. Disclosed is also an atrial appendage occlusion device comprising mesh or braiding of at least one wire or thread, where the occlusion device has been given a shape using a reshaping and/or heat-treatment process, and is self-expandable, as well as configured for safe anchoring in an atrial appendage of the left or right atrium of a heart, comprising proximal retention region at proximal end of the occlusion device; a distal retention region; and a central region between the proximal retention region and the distal retention region; where the occlusion device has a closed distal end without a hub for the wire or thread, and where the proximal retention region is of elongate spherical shape and at least partly hollow, and where the distal retention region comprises a distal anchoring element integrally made of the same mesh or braiding as the hollow elongate spherical proximal retention region. The document also discloses production of an atrial appendage occlusion device, where a spherical hollow mesh or braiding is produced in such a way that thin wires or threads which constitute the finished mesh or braiding are interwoven in the formation of the spherical hollow mesh or braiding at the distal end of the mesh or braiding, so that a distal retention region has a closed shape to the distal end.

Finally there are holes and openings not created by interventional treatment activity, acquired as a result of disease or congenital. Some products for closing acquired or congenital defects are devices having umbrella shaped discs with spikes and a covering cloth. One disc is placed on each side of the defect and then pressed against each other and locked, StarFlex® (NMT Medical Inc®, Boston MA) and CARDIA Patent Foramen Ovale Closure Device® (Cardia Inc®, Burnsville, Minnesota) are such devices. Other devices are made of Nitinol threads, e.g. having a double disc shape with a waist between the discs. They are inserted in openings that are to be closed, one disc on each side of the hole that are to be closed and the waist in the center of the hole, the discs being larger than the hole. There are two examples of such devices. The first, made by Occlutech®, having one fixation point at the end of the device and the second, made by AGA medical® having two fixation points, one at each end of the device. In these devices, the Nitinol threads are joined in the centre of one or both of the discs.

Some of the fixation points have a screw with windings to be attached to a rod. By means of that rod, the devices may be pulled into and pushed out of a catheter when being positioned in the opening to be closed. When in position, the device is detached from the rod by unscrewing the connection. Such fixation points provide a massive aggregation of material preventing access to the interior of the device at the position of the fixation point(s).

A major disadvantage of the prior known devices is that they may not be delivered by a standard over-the-wire technique, also known as the Seldinger technique.

WO2011/093941 discloses a system according to the preamble of claim 1. Thus, there is a need for an improved medical system for delivering an object through a body opening to a target site in a body.

There is also a need for an improved collapsible medical closing device for closing a body opening.

Furthermore, a method of assembling a medical system for delivering an object, performed outside of a body and before using the assembly in any medical procedure would be advantageous.

There is also a need for a simplified method of manufacturing a collapsible medical closing device and enabling delivery of a further device through a hollow sheath and/or enabling delivery of a collapsible medical closing device over a guide wire to remote target sites.

An object of the present disclosure is to provide an improved device and a method for sealing of an opening in a body vessel or the wall of a heart cavity, e.g. a blood vessel or a human heart that is simple to use and that do not involve suturing. Another object of the invention is that all its parts are single use articles.

Furthermore, an improved medical system, which makes medical intervention safer, more reliable, shorter, which system has less risk for infections and less invasive procedures would be advantageous.

### Summary of the Invention

Accordingly, embodiments of the present disclosure preferably seek to mitigate, alleviate or eliminate one or more deficiencies, disadvantages or issues in the art, such as the above-identified, singly or in any combination by providing a closing device, a medical system or a method of assembling a medical system, according to the appended patent claims.

A major disadvantage of the prior known devices is that they may not be delivered by a standard over-the-wire technique, also known as the Seldinger technique. The present disclosure overcomes this disadvantage, amongst others, by providing a closing device that is capable of travelling over a catheter or a guide wire. The disclosed devices are provided with a hole or channel allowing for access into and through the device. Secondly, the disclosed devices have the capacity for a radial retraction.

Embodiments of the present disclosure may be well suited for the selective occlusion of a vessel, lumen, channel, hole, cavity, or the like. Examples, without limitations, are a vessel, lumen, channel, or hole through which blood flows from one vessel to another vessel such as an Atrial Septal Defect (herein after ASD) or a Ventricular Septal Defect (herein after VSD). Other examples could be an Arterial Venous Fistula (AVF), Arterial Venous Malformation (AVM), a Patent Foramen Ovale (PFO), Para-Valvular Leak (PVL), or Patent Ductus Arteriosus (PDA), also called Ductus Botalli.

A medical system according to the invention is recited in claim 1. A method of assembling a medical system according to the invention is recited in claim 14. Preferred embodiments of the invention are recited in the dependent claims.

Some embodiments of the disclosure provide for an improved and simplified method of manufacturing a collapsible medical closing device.

Some embodiments of the disclosure also provide for delivery of further devices through hollow sheath and/or delivery of a collapsible medical closing device over at least one guide wire to remote target sites.

Some embodiments of the disclosure also provide for enabling a collapsible medical closing device to travel over a sheath, such as a catheter inside a body, such as a mammal body, and thus providing a way of positioning an object and sealing a gap of a hole or an opening in a body, such as a mammal body, with one single piece of equipment, i.e. one single system comprising catheters and at least one collapsible medical closing device, which system can be used for both placing an object and sealing a gap.

Some embodiments of the disclosure also provide for enabling an easier procedure of positioning an object inside a body, such as a mammal body and avoiding regular surgery.

Some embodiments of the disclosure also provide for enabling an improved and simplified method of manufacturing a collapsible medical closing device and enabling putting an opening in the center of disc-shaped sections, by making sure that there is no need for joining points in the center of any of the disc-shaped sections.

In one embodiment the thread's two ends are joined by means of welding, however other means of joining the ends may be used, like pinching the ends together, or hooking them together.

Some embodiments of the disclosure also enables a collapsible medical closing device to be fitted to various catheters with different sizes, and thus provides for compact coaxial aggregates without substantially increasing the cross section or diameter of the collapsible medical closing device.

Some embodiments of the disclosure also provide for an easier way of placing an object, like an artificial valve in the aortic valve position and avoiding invasive open heart surgery.

Some embodiments of the disclosure also provide for an alternative of providing a collapsible medical closing device with an opening by the use of fasteners, which device can be customized to fit a catheter of a particular size. Such fasteners can be provided in different sizes so that a collapsible medical closing device can be used for a wide variety of catheters with different sizes. Furthermore, the fasteners can be automatically closable/sealable or self-closing/self-sealing, depending on pressure. The fasteners can also be provided with a through bore.

Some embodiments of the disclosure also provide for enabling a gap of an opening in a body, such as a mammal body, to be sealed with a collapsible medical closing device from both sides of the opening and thus providing a more reliable sealing of an opening in a body.

In some embodiments the collapsible medical closing device has two disc-formed or cylinder-formed sections with an intermediate shaft section. However, the collapsible medical closing device could have any number of disc-formed or cylinder-formed sections with intermediate shaft sections in-between.

Some embodiments of the disclosure also provide for a more reliable sealing of a gap of an opening, such as an opening in a cardiac wall, an opening to a coronary vessel, an opening in a percutaneous delivery channel, an opening in the abdominal wall or an opening to an aneurysm.

Some embodiments of the disclosure also provide for a way of decreasing the size and diameter of the tubular, cylindrical or disc-shaped collapsible medical closing device during delivery of it to a target site in a body, such as a mammal body.

Some embodiments of the disclosure also provide a means of releasing the collapsible medical closing device from its delivery position inside the restraining catheter into its target position at the target site. Releasing may be done in a safe way, allowing for retracting a closing device prior to fully releasing it into the body.

Some embodiments of the disclosure also provide for delivering a medical closing device, such as a collapsible medical closing device by the use of a compact unit or an integrated unit. This compact design may even be provided in combination with delivery of another object passing through the closing device and a tissue opening to a target site before closing off the opening with the closing device.

Some embodiments of the disclosure also provide for a collapsible medical closing device, which is easily deployed.

Some embodiments of the disclosure also make medical intervention safer, more reliable, shorter, and/or lower the risk for infections and decreases the number of invasive procedures.

Some embodiments of the disclosure also provide for cost effective health care.

In one embodiment, the device may be filled with sealing material like polytetrafluorethylen (PTFE). The sealing material may be polyurethane. The sealing material may be polyvinyl or other polymers. The sealing material may be a biological degradable material. The degradable material may be polydioxanone (PDS). The degradable material may be polyglactin (Vicryl). The degradable material may be polyglycolic acid (Dexon). The sealing material may be a resorbable material that will be resorbed by the body. The sealing material may be provided as a filling of the closing device. The sealing material may be arranged inside the closing device. The sealing material may be integrated with the closing device. Integration may be provided as a monolithic unit. The sealing material may be interweaved into threads of the closing device. The sealing material may be provided as a coating covering at least a portion of a surface of the closing device. The sealing material may also comprise nonresorbable cloths or Dacron.

In some embodiments, the Nitinol thread may have the mentioned sealing materials attached to the thread instead, and not freely floating in the disc-shaped sections.

In some embodiments the thread is made of a Magnesium alloy.

In further embodiments, the thread or the wire comprises at least one micro coil. The use of one or several micro coils improves the flexibility of the thread or wire. Furthermore, the use of one or several micro coils has the advantage of making the collapsible medical closing device more dense, since the structure is made more micro-porous and thereby enabling a faster biological closing with thrombocytes, fibrin and cells.

In yet another embodiment an expanding or swelling synthetic material is used, which may expand and thereby contribute to filling the gap in the hole or puncture site.

The swelling material may be a swelling polymer, such as disclosed in WO2009049677, which is incorporated herein in its entirety for all purposes.

An important feature of the collapsible medical closing device here described is that the whole device may change in diameter, one diameter when placed outside a catheter or a rod, and both disc-shaped sections opened, and another smaller diameter when the catheter or rod is retracted from the device. Thus the device will close the opening with a disc on each side of the hole and then retract in the radial direction in the order of pulling tissue towards the device center.

In one embodiment the side of disc-shaped sections facing tissue after deployment may have hooks or barbs in order to increase friction against tissue. Importantly, the device has no central opening in its natural shape, when not being mounted on a catheter or a rod.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the disclosure are capable of will be apparent and elucidated from the following description of embodiments of the present disclosure, reference being made to the accompanying drawings, in which:
Fig. 1 is a lateral view in which a collapsible medical closing device is schematically shown before temperature memory fixation;
Fig.2 is a lateral view of a collapsible medical closing device after temperature memory fixation;
Fig.3a is a lateral view, which illustrates a guide wire passing through the collapsible medical closing device;
Fig.3b is a lateral view, which illustrates a catheter passing through the collapsible medical closing device;
Fig.4a shows a lateral view of a closing device and illustrates a catheter passing through the closing device;
Fig.4b shows a front view of a closing device and illustrates a catheter passing through the closing device;
Fig.5 is a lateral view of the collapsible medical closing device inserted into another catheter;
Fig.6 is a lateral view of a pushing catheter behind the collapsible medical closing device positioned in a catheter, the collapsible medical closing device being pushed halfway out;
Fig.7 is a lateral view of the medical system inside the left ventricle of a heart;
Fig.8 shows another lateral view of the medical system inside the left ventricle of a heart;
Fig.9 is yet another lateral view of the medical system inside the left ventricle of a heart;
Fig.10 is a further lateral view of the medical system inside the left ventricle of a heart;
Fig.11a is an anatomic sketch of the central structures in a human thorax used for description of a Ductus Botalli.
Fig.11b: is an anatomic sketch of the central structures in a human thorax used for description of a closure of a Ductus Botalli by means of a collapsible medical closing device;
Fig. 12a is an anatomic sketch of the central structures in a human thorax used for description of a fistula between a left coronary artery and the pulmonary artery;
Fig. 12b is an anatomic sketch of the central structures in a human thorax used for description of a closure of a fistula between a left coronary artery and the pulmonary artery by means of a closing device;
Fig. 13a shows a section of a human body surface;
Fig. 13b depicts how a treatment hole is closed by means of a collapsible medical closing device;
Fig. 14 is another front view of a collapsible medical closing device;
Fig. 15 is a lateral view of a collapsible medical closing device after temperature memory fixation; and
Fig. 16 is a lateral view of a collapsible medical closing device.

### Description of embodiments

Specific embodiments of the disclosure will now be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to the sealing of an opening in a body and in particular to the sealing of a vessel or a wall of a heart cavity. However, it will be appreciated that the disclosure is not limited to this application but may be applied to many other applications including for example the sealing of an aneurysm or a puncture site.

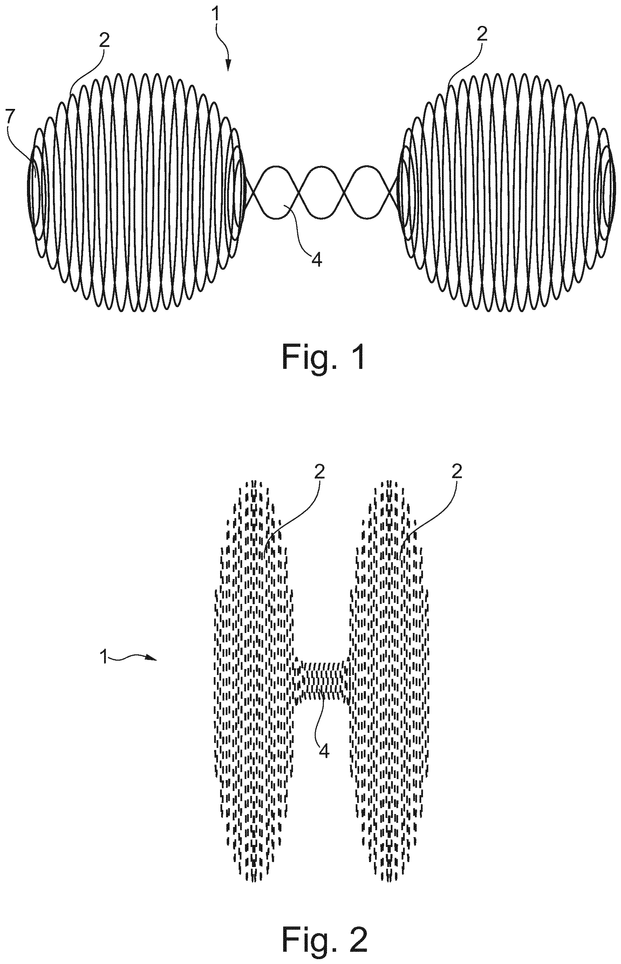

In an embodiment of the disclosure according to Fig. 1 the collapsible medical closing device 1 is provided with two disc-shaped sections 2 with an intermediate shaft section 4. Fig.1 shows a schematic description of the collapsible medical closing device 1 before temperature setting of the definite shape. The collapsible medical closing device 1 consists of two bodies, preferably disc-shaped 2, having a shaft section 4 between them. The whole device can be constructed from one sufficiently long thread 6, wire or fiber that may have a connecting or welding point 7.

Further embodiments of the disclosure are illustrated in Fig. 2-14.

Fig.2 shows the collapsible medical closing device 1 after temperature setting of a definite shape, a shape the device wants to return to, e.g. by a resilient spring effect. The definite shape may be a relaxed, expanded state. The here presented collapsible medical closing device 1 may be made of Nitinol, an alloy of Nickel and Titanium, which is a so called shape memory alloy. Such alloys tend to have a temperature induced phase change, which will cause the material to have a preferred configuration which can be fixed by heating the material above a certain transition temperature to induce a change in the phase of the material. When the alloy is cooled back down, the alloy will "remember" the shape it was in during the heat treatment and will tend to assume that configuration unless constrained from doing so.

However, the collapsible medical closing device 1 may also be made of a Magnesium alloy, or bioresorbable materials like polytetrafluorethylen (PTFE), polyurethane, polyvinyl or other polymers, a biological degradable material like polydioxanone (PDS), polyglactin (Vicryl), polyglycolic acid (Dexon) or other resorbable materials that will be resorbed by the body or other bioabsorbable materials or polymers.

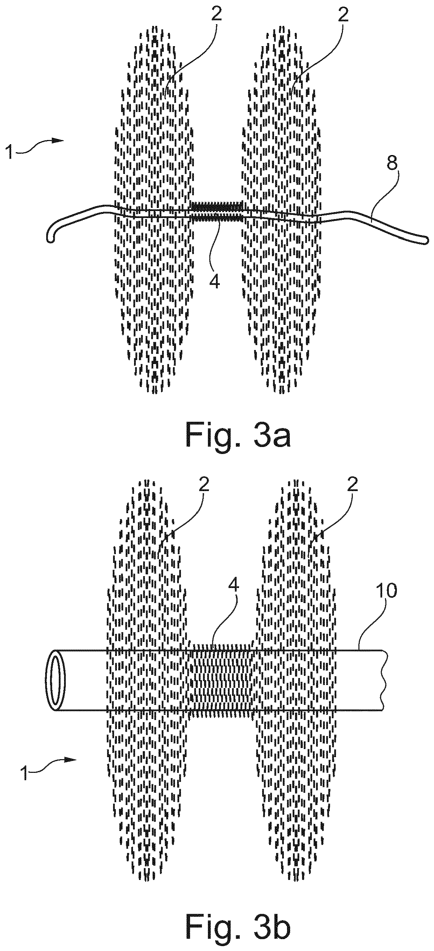

Fig.3a shows the collapsible medical closing device 1 in its definite shape while having a guide wire 8 running through its center. The definite shape may be a relaxed, expanded state of the device 1.

Fig.3b shows the collapsible medical closing device 1 in its definite shape while having a treatment catheter 10 running through its center.

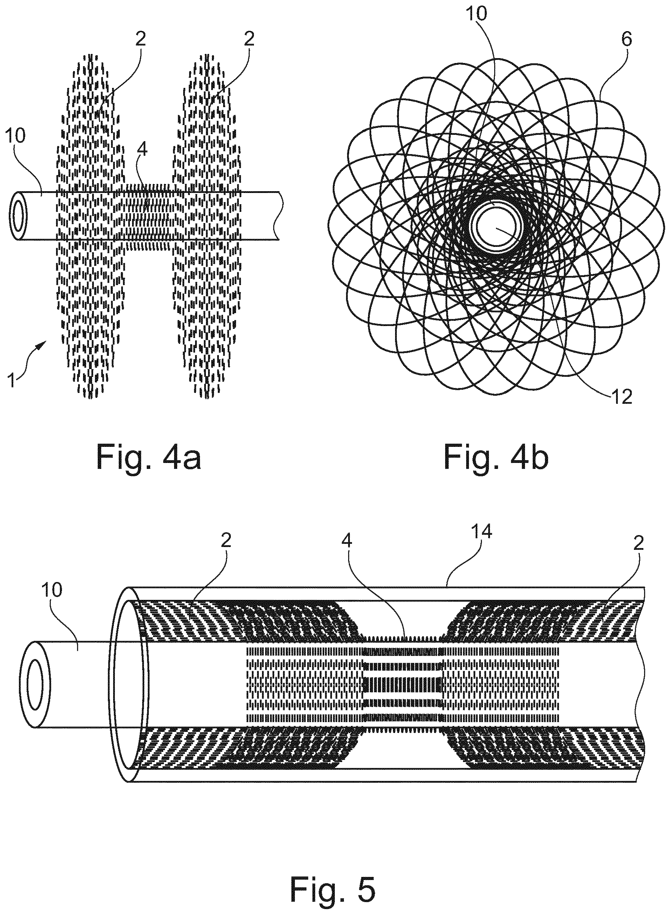

Figs.4a and 4b show the collapsible medical closing device 1 in its definite shape while having a treatment catheter 10 running through its center, Fig. 4a from the side and Fig. 4b seen from the end. An opening or a channel 12 permits a treatment catheter 10 to pass through the center of the device in its longitudinal direction. The opening or channel 12 is not permanent or preset, instead the opening or channel 12 is made while mounting the device on a guide wire or a catheter, respectively.

Fig.5 shows the collapsible medical closing device 1 mounted on a treatment catheter 10 and restrained inside a restraining catheter 14 from the outside. Under other conditions, the collapsible medical closing device 1 might be restrained inside a restraining catheter 14 without a treatment catheter 10 or a guide wire 8 if they are not needed.

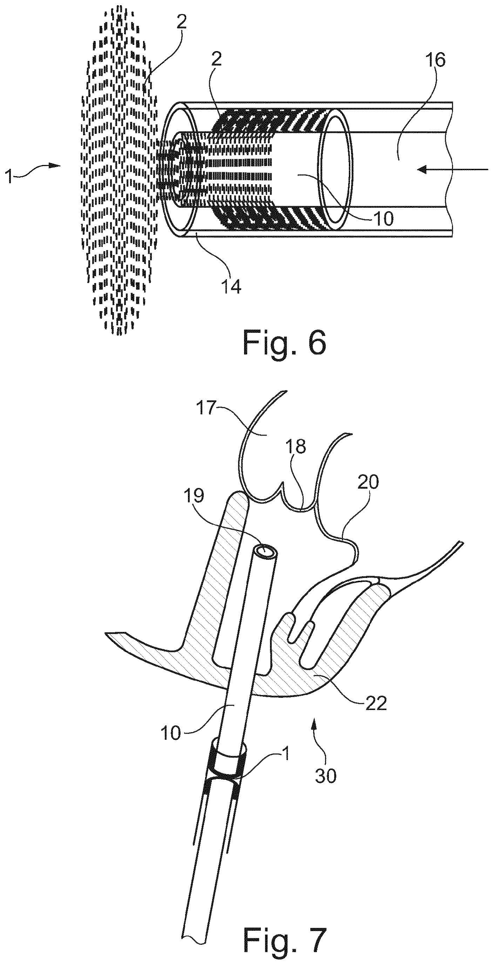

Fig.6 shows the collapsible medical closing device 1 deployed to approximately 50%, in which position its front disc 2 has been released from the restraining catheter 14 by means of a pusher catheter 16 which is positioned between a treatment catheter 10 and a restraining catheter 14.

Fig.7 depicts a situation in a human heart 30 where a treatment catheter 10 is penetrating the left ventricular muscular wall 22. In this case the treatment catheter 10 is aiming for the aortic valve 18, where an object 19, such as an artificial valve may be placed. The catheter 10 makes a large hole in the left ventricular wall 22.

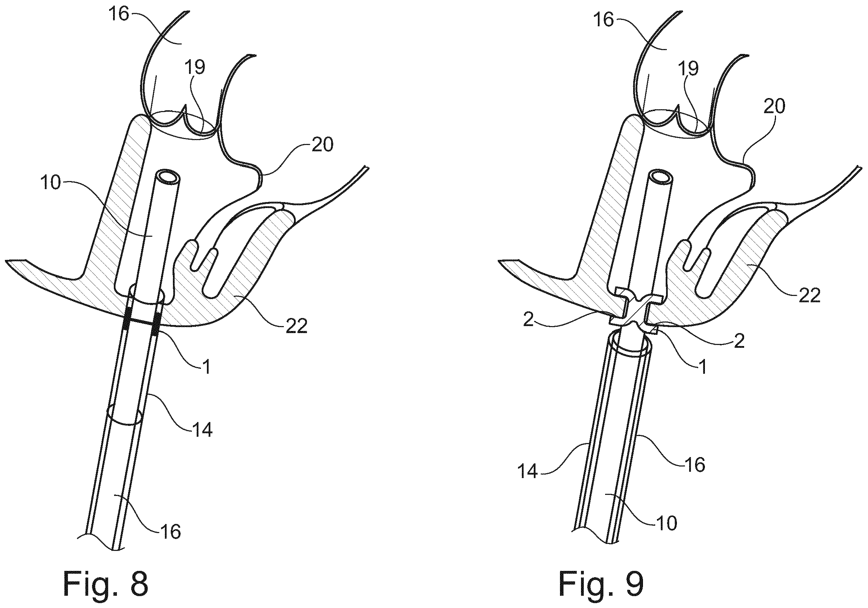

Fig.8 shows a situation where the object 19 has been placed in the aortic valve position. The treatment catheter 10 is empty. The restraining catheter 14 with the collapsible medical closing device 1 is advanced over the treatment catheter 10, through the hole in the left ventricular wall 22.

Fig.9 shows a situation where the collapsible medical closing device 1 has been released by means of pushing the pushing catheter 16 forward, now one disc-shaped section 2 on the inside of the left ventricle and one disc-shaped section 2 on the outside of the left ventricle. The treatment catheter 10 is still in a position inside the ventricle, running through the shaft section 4 of the collapsible medical closing device 1.

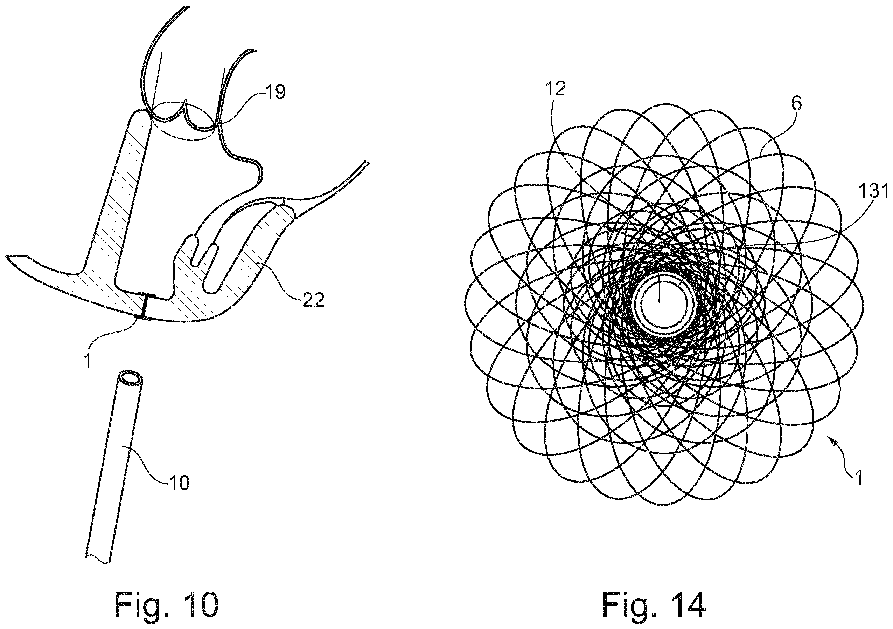

Fig.10 shows a situation where the treatment catheter 10 has been retracted from within the left ventricle and the central part of the collapsible medical closing device 1. The two disc-shaped sections 2 are hugging the left muscular ventricular wall 22 with one disc-shaped section 2 on the inside and one disc-shaped section 2 on the outside of the wall 22. As the treatment catheter 10 has been retracted from the shaft 4, the shaft section 4 is now returning to its preset definite position. While the shaft section 4 retracts to its preset shape, the collapsible medical closing device 1 will contract radially and close the hole. If filling material is added inside the collapsible medical closing device 1, it will additionally contribute to a blood tight sealing of the hole.

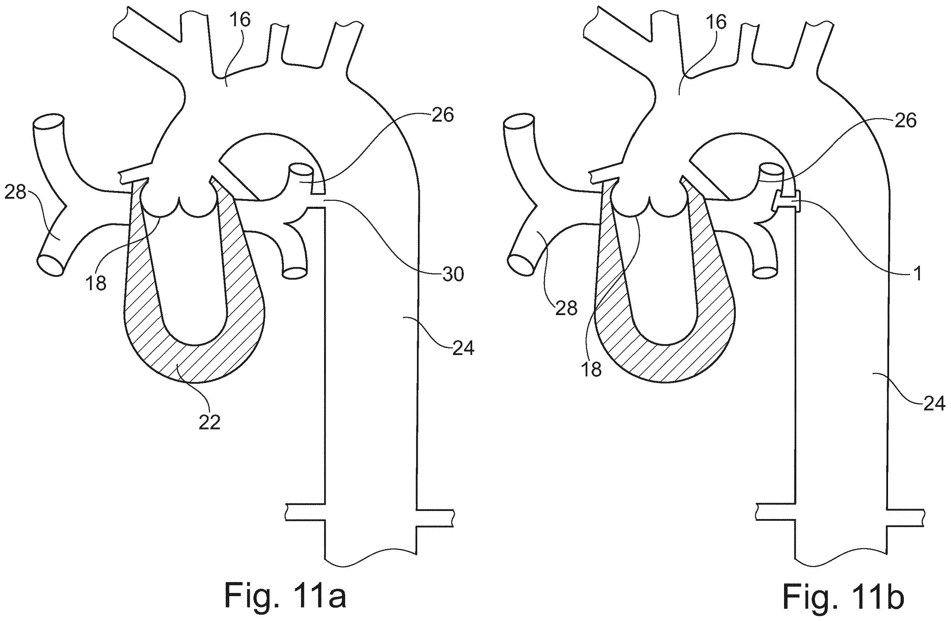

Fig. 11a is an anatomic sketch of the central structures in a human thorax, in which the heart and the large central vessels are shown. In Fig. 11a the ascending aorta 16, the aortic valve 18, the left ventricular wall 22, the descending aorta 24, the left pulmonary artery 26 and the right pulmonary artery 28 are shown. In a position between the descending aorta 24 and the left pulmonary artery 26, the Ductus Botalli is located. Ductus Botalli allows blood flow between the pulmonary circulation and the peripheral circulation allowing arterial and venous blood mixture until a fetus is born. This mixture is necessary since a fetus has no functioning lungs. The Botalli is supposed to close during the first days after birth, however, in some congenital cases it does not. An open Ductus Botalli allowing mixture and backward flow into the pulmonary system is life threatening. Surgical closure has been the method of choice until recently. Closure by means of catheter is nowadays practiced, however, such catheter treatment is difficult with the prior art closing devices that does not allow a guide wire to pass through the closing devices.

Fig. 11b depicts a situation where a Ductus Botalli has been closed by means of a collapsible medical closing device 1 according to this disclosure.

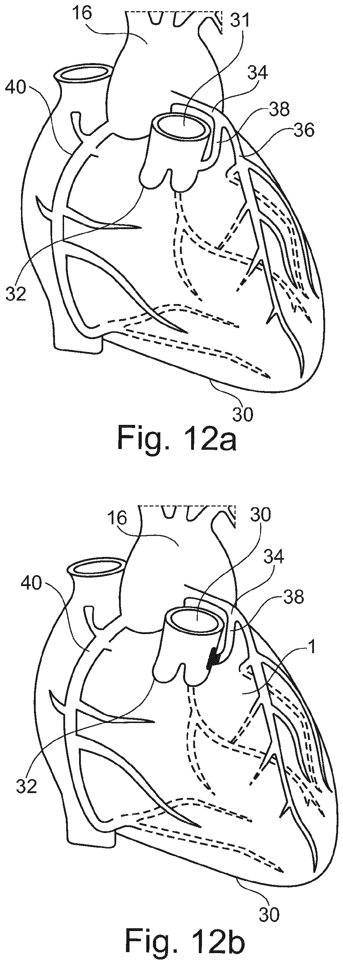

Fig. 12a shows another congenital condition, when a child is born with an abnormal connection between a coronary artery and a pulmonary artery, such connections may result in heart failure. In Fig. 12, 30 is the human heart, 31 the main pulmonary artery, 32 the pulmonary valve, 34 the left coronary artery main stem, 36 the left anterior descending coronary artery and 40 the right coronary artery main stem. An abnormal connection, an arterio-venous fistula 38 is shown allowing blood flow between the coronary artery and the pulmonary artery. Surgical closure has been the method of choice until recently. Closure by means of catheter is nowadays practiced, however, such catheter treatment is difficult with the prior art closing devices that does not allow a guide wire to pass through the closing devices.

Fig.12b depicts a situation where an abnormal arterio-venous fistula 38 has been closed by means of a collapsible medical closing device 1 according to this disclosure. In this embodiment, the closing device 1 has only a single disc shaped section 2 in its expanded state. Alternatively, the second disc shaped portion 2 may be restricted by the tissue of the fistula 38. The resilient outward tension against the lumen wall may provide for a particular advantageous anchoring of the device.

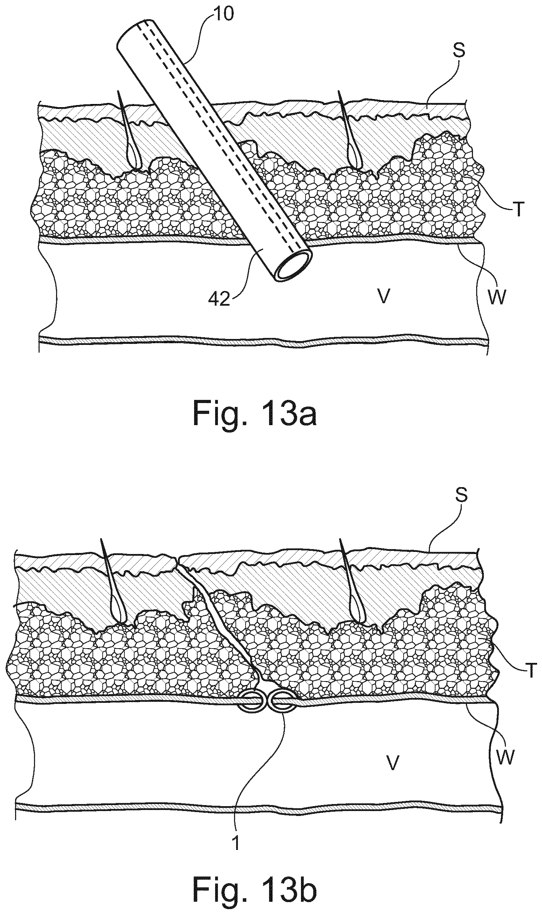

Fig.13a shows a section of a human body surface. Under the skin S, subcutaneous tissue T is located above a vessel V. A treatment catheter 10 is penetrating the skin S and the subcutaneous tissue T, allowing access to a vessel V below, through a vessel wall W. The puncture hole at the puncture site 42 will induce bleeding between the layers if the treatment catheter 10 is withdrawn without closing the hole in the vessel.

Fig.13b depicts how such a treatment hole 42 is closed by means of a collapsible medical closing device 1 according to this disclosure. The puncture is reliably sealed off, preventing bleeding from the vessel V through the skin S. the device 1 is implanted into the wall W while the tissue closes off resiliently or assisted by e.g. a surgical topical patch or tape.

In another embodiment of the disclosure according to

Fig. 14, which is a front view of a collapsible medical closing device 1, the collapsible medical closing device 1 comprises a fastener 131, which may be shaped as a collar, with a central opening 12.

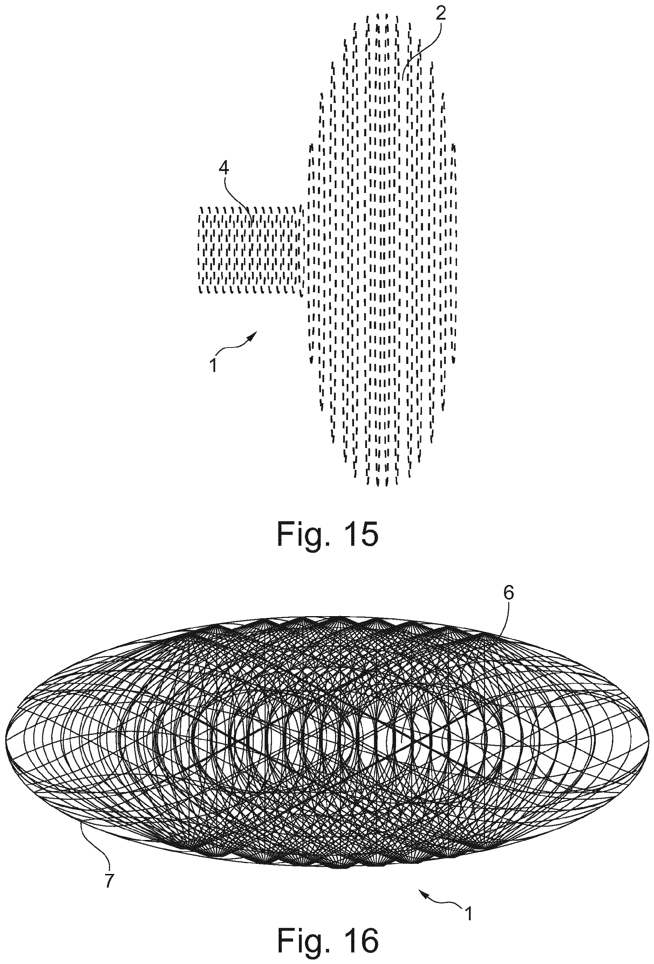

In yet another embodiment of the disclosure according to Fig. 15, which is a lateral view of a collapsible medical closing device 1 after temperature memory fixation, the collapsible medical closing device 1 is provided with only one disc-shaped section 2 and an intermediate shaft section 4.

In a further embodiment of the disclosure according to Fig. 16, which is a lateral view of a collapsible medical closing device 1, the collapsible medical closing device 1 is provided with one section, which is shaped like a rugby ball, i.e. the section has an oval shape. Also in this embodiment, the collapsible medical closing device 1 can be constructed from one sufficiently long thread 6, wire or fiber that may have a connecting or welding point 7 and allow an object like a guide wire or a catheter to pass through its structure.

One embodiment of this disclosure is a method for closing a hole in a gap or a hole in a body vessel or a heart using a collapsible medical closing device 1, which comprises the step of placing the collapsible medical closing device 1 outside of a treatment catheter 10 which is used to treat a medical condition. The collapsible medical closing device 1 is then restrained outside of the treatment catheter by means of a second catheter 14 before the treatment catheter is taken into use. Such mounting of a collapsible medical closing device 1 may be done completely during fabrication of the collapsible medical closing device 1 and before sterilization and packing before use, in order to simplify its use. During surgery, when the treatment, e.g. placing of an artificial heart valve 19, is completed, the collapsible medical closing device 1 is advanced over the treatment catheter inside the restraining catheter until the front disc 2 is inside of the opening to be closed, then the restraining catheter 14 is retracted to unfold the disc-shaped sections 2 in the heart chamber inside of the opening, with the treatment catheter 10 still in the hole. The restraining catheter 14 is further retracted to allow the second disc 2 to unfold outside the hole. Now the treatment catheter 10 may be retracted. Immediately, the two disc-shaped sections 2 and the shaft section 4 will retract from a larger diameter to a smaller diameter of the disc-shaped sections and the shaft section 4, returning to its original shape it had before being placed outside of the treatment catheter 10, closing the gap in the hole. This retraction from a large to a smaller diameter after attachment of the disc-shaped sections 2 outside and inside of the hole will close the hole in the center while the collapsible medical closing device 1 will return to its original shape, i.e. the shape it had before a central channel 12 was made in the collapsible medical closing device 1.

In another embodiment the collapsible medical closing device 1 may also be used over a guide wire 8 only, and inside a restraining catheter 14. Thus the collapsible medical closing device 1 may easily travel long distances inside the body, tracking a guide wire 8 to places that are difficult to access, i.e. where the state of art devices used nowadays may not reach, since they may not track over guide wires. Such an important treatment possibility is the closure of fistulas, e.g. coronary artery fistulas. Another application of the device is to use it as a vascular plug, closing vessels, or in another important application to close leaks outside of artificial heart valves, so-called paravalvular leaks.

In yet another embodiment, a method is disclosed, which method is for delivering an object through a body opening to a target site in a body. In this method an object 19 is positioned inside a treatment catheter 10 and the treatment catheter 10 is positioned inside a collapsible medical closing device 1. Thereafter the treatment catheter 10 is inserted together with the object 19 and the collapsible medical closing device 1 into the body. A distal end of said treatment catheter 10 and the collapsible medical closing device 1 are positioned at the target site inside the body opening. The object 19 is delivered to the target site within the body through the treatment catheter 10. The collapsible medical closing device 1 is delivered to the opening for closing the latter; and the treatment catheter 10 is removed from the body. This enables the collapsible medical closing device 1 to travel over a catheter or a guide wire inside a mammal body and thus provides a way of placing an object and sealing a gap of an opening in a body, such as a mammal body with one single piece of equipment, i.e. one single system comprising catheters and closing device.

In one embodiment the target site is remote from the body opening and the object 19 delivered may be an artificial valve for an aortic valve position, a coronary stent for implanting in a coronary vessel, a percutaneous catheter, an aneurysm filling unit, a surgical instrument, or an intubation tube.

This way a simplified procedure for placing an artificial valve in the aortic valve position is enabled and open heart surgery may be avoided.

In another embodiment the collapsible medical closing device 1 is restrained by inserting the collapsible medical closing device 1 into a restraining catheter 14 prior to the insertion of the treatment catheter 10.

This provides a way of decreasing the size and diameter of the tubular, cylindrical or disc-shaped collapsible medical closing device 1 during delivery of it to a desired position in a body, such as a mammal body.

In yet another embodiment a pushing catheter 16 is positioned inside the restraining catheter 14 adjacent to the collapsible medical closing device 1, further away from the target site than the collapsible medical closing device 1, thus providing a means of releasing said collapsible medical closing device from its delivery position inside the restraining catheter.

In another embodiment the restraining catheter 14, the pushing catheter 16, the object 19, the treatment catheter 10 and said collapsible medical closing device 1 are inserting together into the body.

In yet another embodiment the collapsible medical closing device 1 is pushed over the treatment catheter 10 with the pushing catheter 16 until the collapsible medical closing device 1 has been released, so that a first disc-formed or cylinder-formed section 2 of the collapsible medical closing device 1 is positioned on an inside of a gap to be sealed. The pushing catheter may thereafter be removed. Then the restraining catheter 14 may be removed so that the first disc-formed or cylinder-formed section 2 of the collapsible medical closing device 1 is positioned on the inside of the gap to be sealed with the collapsible medical closing device 1 and a second disc-formed or cylinder-formed section 2 is positioned on an outside of the gap, whereby a shaft section 4 of the collapsible medical closing device 1 is returned to its preset shape and the collapsible medical closing device 1 thereby is radially contracted so as to close said gap.

This enables a gap of an opening in a body, such as a mammal body to be sealed with the collapsible medical closing device 1 from both sides of the opening and thus provides a more reliable sealing of an opening in a body, such as a mammal body.

The gap may be a gap of an opening, such as an opening in a cardiac wall, e.g. in apex of heart muscle, an opening to a coronary vessel, an opening in a percutaneous delivery channel, an opening in an abdominal wall, e.g. for laparoscopy or an opening to an aneurysm, whereby clogging material may be delivered.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The present disclosure has been described above with reference to specific embodiments. However, other embodiments than the above described are equally possible within the scope of the disclosure. Different method steps or a different order thereof than those described above, may be provided within the scope of the disclosure. The different features and steps of the disclosure may be combined in other combinations than those described. The scope of the disclosure is only limited by the appended patent claims.

## Claims

1. A medical system for delivering an object through a body opening to a target site in a body, comprising:
a treatment catheter (10); and
a collapsible medical closing device (1) for substantially closing said body opening, said collapsible medical closing device (1) comprising:
a network of at least one thread (6), wire or fiber, and
a closeable through channel in said network having an non-permanent opening (12) for receiving said treatment catheter (10) therein for delivery of said collapsible medical closing device (1) over said treatment catheter (10), **characterised in that** said collapsible medical closing device (1) is radially self-contractible to occlude said body opening, when said treatment catheter (10) is retracted.

2. The medical system of claim 1, wherein said treatment catheter (10) is adapted for delivering an object (19).

3. The medical system of any of claims 1-2, wherein said thread (6), wire or fiber has a connection or a welding point (7).

4. The medical system of claim 3, wherein said treatment catheter (10) is positioned coaxially inside said non-permanent opening (12) of said collapsible medical closing device (1).

5. The medical system of any of claims 1-4, wherein a size, such as a diameter, of said non-permanent opening (12) is adjustable as desired.

6. The medical system of any of claims 1-5, further comprising a fastener (131), said fastener (131) being shaped as a collar, with a central opening, wherein said central opening forms said non-permanent opening (12).

7. The medical system of any of claims 1-6, wherein said collapsible medical closing device (1) further comprises at least one disc-formed or cylinder-formed section (2), being connected to a shaft section (4).

8. The medical system of any of claims 1-7, wherein said collapsible medical closing device (1) is adapted for sealing a gap of an opening, such as an opening in a cardiac wall, an opening to a coronary vessel, an opening in a percutaneous delivery channel, an opening in an abdominal wall or an opening to an aneurysm.

9. The medical system of any of claims 1-8, further comprising a restraining catheter (14) for restraining said collapsible medical closing device (1) during delivery; said restraining catheter (14) being slidable around said collapsible medical closing device (1) while enclosing said collapsible medical closing device (1).

10. The medical system of claim 9, further comprising a pushing catheter (16) for releasing said collapsible medical closing device (1), said pushing catheter (16) being positioned inside said restraining catheter (14), said pushing catheter (16) being capable of pushing said collapsible medical closing device (1) through said restraining catheter (14) until said collapsible medical closing device (1) is released from said restraining catheter (14), wherein said pushing catheter (16) is preferably positioned coaxially inside said restraining catheter (14).

11. The medical system of any of claims 1-10, comprising a safety wire releasably connected to said collapsible medical closing device (1) for retrieving said collapsible medical closing device (1) upon delivery.

12. The medical system of any of claims 1-11, wherein said thread (6), wire or fiber is spun out of one single thread, wire or fiber, wherein said thread (6), wire or fiber have a first end and a second end and wherein only said first and said second end of said thread, wire or fiber are joined.

13. The medical system of any of claims 1-12, wherein said thread (6) or wire comprises at least one micro coil.

14. A method of assembling a medical system for delivering an object (19) **characterized by:**
positioning a treatment catheter (10) and optionally a guide wire (8) through the non-permanent opening (12) of said collapsible medical closing device (1) of claim 1;
positioning said collapsible medical closing device (1) and said treatment catheter (10) inside a restraining catheter (14); and positioning a pushing catheter (16) inside said restraining catheter (14) adjacent to the collapsible medical closing device (1),
wherein said method is performed outside of a body and before using said assembly in any medical procedure.

## Patentansprüche

1. Medizinisches System zur Zuführung eines Objekts durch eine Körperöffnung zu einer Zielstelle in einem Körper, umfassend:
einen Behandlungskatheter (10); und
eine zusammenklappbare medizinische Schließvorrichtung (1), um die Körperöffnung im Wesentlichen zu verschleißen, wobei die zusammenklappbare medizinische Schließvorrichtung (1) umfasst:
ein Geflecht aus mindestens einem Faden (6), einem Draht oder einer Faser, und
einen verschließbaren Durchgangskanal in dem Geflecht, der eine nicht-permanente Öffnung (12) zur Aufnahme des Behandlungskatheters (10) in demselben umfasst, um die zusammenklappbare medizinische Schließvorrichtung (1) über den Behandlungskatheter (10) zuführen zu können, **dadurch gekennzeichnet, dass** sich die Schließvorrichtung (1) radial selbst zusammenziehen kann, um die Körperöffnung zu okkludieren, wenn der Behandlungskatheter (10) zurückgezogen wird.

2. Medizinisches System gemäß Anspruch 1, wobei der Behandlungskatheter (10) für die Zuführung eines Objekts (19) angepasst ist.

3. Medizinisches System gemäß einem beliebigen der Ansprüche 1-2, wobei der Faden (6), der Draht oder die Faser einen Verbindungspunkt oder eine Schweißstelle (7) aufweist.

4. Medizinisches System gemäß Anspruch 3, wobei der Behandlungskatheter (10) koaxial innerhalb der nicht-permanenten Öffnung (12) der zusammenklappbaren medizinischen Schließvorrichtung (1) positioniert ist.

5. Medizinisches System gemäß einem beliebigen der Ansprüche 1-4, wobei sich eine Größe, wie beispielsweise ein Durchmesser, der nicht-permanenten Öffnung (12) je nach Bedarf anpassen lässt.

6. Medizinisches System gemäß einem beliebigen der Ansprüche 1-5, ferner ein Befestigungsmittel (131) umfassend, wobei das Befestigungsmittel (131) als eine Schelle mit einer zentralen Öffnung geformt ist, wobei die zentrale Öffnung die nicht-permanente Öffnung (12) bildet.

7. Medizinisches System gemäß einem beliebigen der Ansprüche 1-6, wobei die zusammenklappbare medizinische Schließvorrichtung (1) ferner mindestens einen scheibenförmigen oder zylinderförmigen Abschnitt (2) umfasst, der mit einem Wellenabschnitt (4) verbunden ist.

8. Medizinisches System gemäß einem beliebigen der Ansprüche 1-7, wobei die zusammenklappbare medizinische Schließvorrichtung (1) angepasst ist, um eine Lücke einer Öffnung, wie beispielsweise einer Öffnung in einer Herzwand, einer Öffnung zu einem Herzkranzgefäß, einer Öffnung in einem perkutanen Zuführkanal, einer Öffnung in einer Abdominalwand oder einer Öffnung zu einem Aneurysma zu verschließen.

9. Medizinisches System gemäß einem beliebigen der Ansprüche 1-8, ferner einen Rückhaltekatheter (14) zur Rückhaltung der zusammenklappbaren medizinischen Schließvorrichtung (1) während der Zuführung umfassend; wobei sich der Rückhaltekatheter (14) um die zusammenklappbare medizinische Schließvorrichtung (1) herum verschieben lässt, währen er die zusammenklappbare medizinische Schließvorrichtung (1) umschließt.

10. Medizinisches System gemäß Anspruch 9, ferner einen Schiebekatheter (16) zur Freigabe der zusammenklappbaren medizinischen Schließvorrichtung (1) umfassend, wobei der Schiebekatheter (16) innerhalb des Rückhaltekatheters (14) positioniert ist, wobei der Schiebekatheter (16) in der Lage ist, die zusammenklappbare medizinische Schließvorrichtung (1) durch den Rückhaltekatheter (14) zu schieben, bis die zusammenklappbare medizinische Schließvorrichtung (1) von dem Rückhaltekatheter (14) freigegeben wird, wobei der Schiebekatheter (16) vorzugsweise koaxial innerhalb des Rückhaltekatheters (14) positioniert ist.

11. Medizinisches System gemäß einem beliebigen der Ansprüche 1-10, einen Sicherheitsdraht umfassend, der auf freigebbare Weise mit der zusammenklappbaren medizinischen Schließvorrichtung (1) verbunden ist, um die zusammenklappbare medizinische Schließvorrichtung (1) bei erfolgter Zuführung zurückzuholen.

12. Medizinisches System gemäß einem beliebigen der Ansprüche 1-11, wobei der Faden (6), der Draht oder die Faser aus einem einzelnen Faden, einem einzelnen Draht oder einer einzelnen Faser gesponnen ist, wobei der Faden (6), der Draht oder die Faser ein erstes Ende und ein zweites Ende aufweisen, und wobei nur das erste und das zweite Ende des Fadens, des Drahts oder der Faser verbunden sind.

13. Medizinisches System gemäß einem beliebigen der Ansprüche 1-12, wobei der Faden (6) oder Draht mindestens eine Mikrospule umfasst.

14. Verfahren zum Zusammenbau eines medizinischen Systems zur Zuführung eines Objekts (19), **gekennzeichnet durch:**
die Positionierung eines Behandlungskatheters (10) und optional eines Führungsdrahtes (8) durch die nicht-permanente Öffnung (12) der zusammenklappbaren medizinischen Schließvorrichtung (1) gemäß Anspruch 1;
die Positionierung der zusammenklappbaren medizinischen Schließvorrichtung (1) und des Behandlungskatheters (10) innerhalb des Rückhaltekatheters (14); und die Positionierung eines Schiebekatheters (16) innerhalb des Rückhaltekatheters (14) benachbart zur zusammenklappbaren medizinischen Schließvorrichtung (1),
wobei das Verfahren außerhalb eines Körpers sowie vor Benutzung der Anordnung in einer medizinischen Prozedur durchgeführt wird.

## Revendications

1. Système médical destiné à délivrer un objet à travers une ouverture corporelle sur un site cible dans un corps, comprenant :
un cathéter de traitement (10) ; et
un dispositif de fermeture médicale rétractable (1) destiné à fermer globalement ladite ouverture corporelle, ledit dispositif de fermeture médicale rétractable (1) comprenant :
un réseau d'au moins un câble (6), fil ou fibre, et
un canal traversant fermable dans ledit réseau ayant une ouverture non permanente (12) destinée à recevoir ledit cathéter de traitement (10) en son sein pour une délivrance dudit dispositif de fermeture médicale rétractable (1) le long dudit cathéter de traitement (10), **caractérisé en ce que** ledit dispositif de fermeture médicale rétractable (1) peut se contracter de lui-même de manière radiale pour occlure ladite ouverture corporelle, lorsque ledit cathéter de traitement (10) est rétracté.

2. Système médical selon la revendication 1, dans lequel ledit cathéter de traitement (10) est conçu pour délivrer un objet (19).

3. Système médical selon l'une quelconque des revendications 1 et 2, dans lequel ledit câble (6), fil ou fibre comporte un raccord ou un point de soudure (7).

4. Système médical selon la revendication 3, dans lequel ledit cathéter de traitement (10) est positionné de manière coaxiale à l'intérieur de ladite ouverture non permanente (12) dudit dispositif de fermeture médicale rétractable (1).

5. Système médical selon l'une quelconque des revendications 1 à 4, dans lequel une dimension, telle qu'un diamètre, de ladite ouverture non permanente (12) est réglable comme voulu.

6. Système médical selon l'une quelconque des revendications 1 à 5, comprenant en outre un dispositif de fixation (131), ledit dispositif de fixation (131) étant façonné sous la forme d'un collier, avec une ouverture centrale, où ladite ouverture centrale forme ladite ouverture non permanente (12).

7. Système médical selon l'une quelconque des revendications 1 à 6, dans lequel ledit dispositif de fermeture médicale rétractable (1) comprend en outre au moins une section en forme de disque ou en forme de cylindre (2), qui est reliée à une section de tige (4).

8. Système médical selon l'une quelconque des revendications 1 à 7, dans lequel ledit dispositif de fermeture médicale rétractable (1) est conçu pour obturer un espace d'une ouverture, telle qu'une ouverture dans une paroi cardiaque, une ouverture sur un vaisseau coronarien, une ouverture dans un canal de délivrance percutanée, une ouverture dans une paroi abdominale ou une ouverture sur un anévrisme.

9. Système médical selon l'une quelconque des revendications 1 à 8, comprenant en outre un cathéter de retenue (14) destiné à retenir ledit dispositif de fermeture médicale rétractable (1) pendant la délivrance ; ledit cathéter de retenue (14) pouvant glisser autour dudit dispositif de fermeture médicale rétractable (1) tout en encerclant ledit dispositif de fermeture médicale rétractable (1).

10. Système médical selon la revendication 9, comprenant en outre un cathéter de poussée (16) destiné à libérer ledit dispositif de fermeture médicale rétractable (1), ledit cathéter de poussée (16) étant positionné à l'intérieur dudit cathéter de retenue (14), ledit cathéter de poussée (16) étant en mesure de pousser ledit dispositif de fermeture médicale rétractable (1) à travers ledit cathéter de retenue (14) jusqu'à ce que ledit dispositif de fermeture médicale rétractable (1) soit libéré hors du cathéter de retenue (14), où ledit cathéter de poussée (16) est de préférence positionné de manière coaxiale à l'intérieur dudit cathéter de retenue (14).

11. Système médical selon l'une quelconque des revendications 1 à 10, comprenant un fil de sûreté relié de manière libérable audit dispositif de fermeture médicale rétractable (1) destiné à récupérer ledit dispositif de fermeture médicale rétractable (1) lors de la délivrance.

12. Système médical selon l'une quelconque des revendications 1 à 11, dans lequel ledit câble (6), fil ou fibre est constitué d'un seul câble, fil ou fibre, où ledit câble (6), fil ou fibre présente une première extrémité et une deuxième extrémité et où seules ladite première et ladite deuxième extrémités dudit câble, fil ou fibre sont reliées.

13. Système médical selon l'une quelconque des revendications 1 à 12, où ledit câble (6) ou fil comprend au moins une microbobine.

14. Procédé d'assemblage d'un système médical destiné à délivrer un objet (19) **caractérisé par** :
le fait de positionner un cathéter de traitement (10) et optionnellement un câble de guidage (8) à travers l'ouverture non permanente (12) dudit dispositif de fermeture médicale rétractable (1) selon la revendication 1 ;
le fait de positionner ledit dispositif de fermeture médicale rétractable (1) et ledit cathéter de traitement (10) à l'intérieur d'un cathéter de retenue (14) ; et le fait de positionner un cathéter de poussée (16) à l'intérieur dudit cathéter de retenue (14) de manière adjacente au dispositif de fermeture médicale rétractable (1),
où ledit procédé est réalisé à l'extérieur d'un corps et avant utilisation dudit ensemble dans le cadre d'une quelconque procédure médicale.
